# EUROPÄISCHE PATENTSCHRIFT

(11) **EP 1 207 821 B1**
(45) Veröffentlichungstag und Bekanntmachung des Hinweises auf die Patenterteilung: **04.08.2004**
(21) Anmeldenummer: 99946052.0
(22) Anmeldetag: 27.08.1999
(51) Int. Cl.: A61F 2/44

(54) **ZWISCHENWIRBEL-IMPLANTAT**
INTERVERTEBRAL IMPLANT
IMPLANT INTERVERTEBRAL

(43) Veröffentlichungstag der Anmeldung: 29.05.2002
(73) Patentinhaber: SYNTHES AG Chur, 7002 Chur (CH)
(72) Erfinder: SCHÄR, Manuel, CH-4132 Muttenz (CH); BERNHARD, Jérôme, CH-8001 Zürich (CH); TAGWERKER, Konrad, CH-4053 Basel (CH)
(74) Vertreter: Lusuardi, Werther Giovanni, Dr.
(86) Internationale Anmeldenummer: PCT/EP1999/006332
(87) Internationale Veröffentlichungsnummer: WO 2001/015637

(56) Entgegenhaltungen:
- FR-A- 2 724 312
- FR-A- 2 733 413
- US-A- 4 960 818
- US-A- 5 192 327

## Beschreibung

Die Erfindung betrifft ein Zwischenwirbel-Implantat gemäss dem Oberbegriff des Patentanspruchs 1.
Aus der DE-A 196 15 938 ist ein Zwischenwirbel-Implantat bekannt, welches aus einer langgestreckten Metallplatte hergestellt wird, so dass es röntgenstrahlenundurchlässig ist. Der Nachteil dieses bekannten Implantates besteht darin, dass es röntgenopak ist und somit nach erfolgter Implantation keine Observation der beabsichtigten Wirbelkörperfusion mehr zulässt.
Aus der US-A-5 192 327 BRANTIGAN ist ein gattungsmässiges Zwischenwirbel-Implantat bekannt, welches aus einem karbonfaserverstärkten Kunststoff gefertigt sein kann. Der Nachteil dieses Implantates liegt darin, dass durch die Verstärkung des Kunststoffes das elastische Verhalten des Implantates von demjenigen des umliegenden Knochens abweicht.
Hier will die Erfindung Abhilfe schaffen. Der Erfindung liegt das Problem zugrunde, ein Zwischenwirbel-Implantat zu schaffen, welches sowohl eine postoperative Observation mittels Röntgenstrahlen erlaubt als auch gleichzeitig eine hohe Biokompatibilität aufweist.
Die Erfindung löst die gestellte Aufgabe mit einem Zwischenwirbelimplantat, welches die Merkmale des Anspruchs 1 aufweist. Der vergleichsweise geringe E-Modul des röntgenstrahlendurchlässigen Materials fördert dabei ein optimales Knochenwachstum an das Implantat.

Als röntgenstrahlendurchlässige Materialien eignen sich z.B. Polyetheretherketone (PEEK), ultrahochmolekulare Polyethylene (UHMWPE) oder Polysulfone (PSU), insbesondere solche mit einem E-Modul von 3 bis 5 GPa.

Eine bevorzugte Weiterbildung besteht darin, dass das Zwischenwirbel-Implantat mindestens einen Marker aus einem röntgenopaken Material ausweist, der höchstens 5 Volumenprozente des Zwischenwirbel-Implantats ausmacht. Damit lässt sich im Röntgenbild neben dem neu gebildeten Knochen dennoch die Position des Implantats ausmachen. Die Marker bestehen vorzugsweise aus Titan oder Tantal in Form von Stiftchen oder Kügelchen.

Eine bevorzugte Weiterbildung besteht darin, dass die Deck- und Grundflächen mit einer dreidimensionalen Strukturierung versehen sind, vorzugsweise in regelmässiger Anordnung, z.B. in Form von kreissegmentförmig angeordneten Zähnen.
Vorzugsweise verlaufen die Deck- und Grundflächen keilförmig zueinander, z.B. in einem Winkel von 10°- 20°.

Eine bevorzugte Weiterbildung besteht darin, die Deckfläche und/oder die Grundfläche mit einer oder mehreren Führungsnuten zu versehen, welche gegen die Hohlzylinderachse gerichtet sind. Die Führungsnuten sind dabei vorzugsweise - von der Hohlzylinderachse (6) aus gesehen - um einen Winkel von 45° ± 15° angeordnet.

Das Verhältnis DF/FQF zwischen der Deckfläche DF und der freien Querschnittsfläche FQF des von der inneren Mantelfläche umschlossenen Hohlzylinders sollte zweckmässigerweise im Bereich von 0,5 bis 1,6 liegen.

Eine weitere bevorzugte Weiterbildung besteht darin, dass die Hohlzylinderwand eine gegen die Hohlzylinderachse gerichtete, von der Deckfläche zur Grundfläche reichende Einbuchtung aufweist. Die Hohlzylinderwand weist dabei vorzugsweise an ihrer höheren Seite einen parallel zur Hohlzylinderachse verlaufenden, von der Deckfläche zur Grundfläche reichenden Trennschlitz auf, der das Implantat U-förmig gestaltet. Die Einbuchtung wird vorzugsweise, von der Hohlzylinderachse aus gesehen, an der dem Trennschlitz gegenüberliegenden Seite der Hohlzylinderwand angebracht.
Die posteriore Einbuchtung gewährleistet eine optimale Auflage des Implantats auf der Wirbelkörperendplatte. Damit wird eine hohe Primärstabilität erreicht. Durch die damit erzielte Passgenauigkeit wird ein laterales Wegrutschen des Implantats erschwert.
Der Trennschlitz trägt dazu bei, dass der an den Wirbelkörperendplatten angelagerte Knochen möglichst schnell von anterior her in das Implantat einwachsen kann. Im weiteren erlaubt der Trennschlitz ein Füllen (z.B. mit Knochenspänen) in situ. Der Trennschlitz ist vorzusweise 6 bis 10 mm breit.

Eine weitere bevorzugte Weiterbildung besteht darin, dass die äussere Mantelfläche mit einer oder mehreren Haltenuten versehen ist, welche quer zur Hohlzylinderachse verlaufen und den gleichen Abstand zur Deck- und Grund-fläche aufweisen. Vorzugsweise sind mehrere Haltenuten vorgesehen, welche von der Einbuchtung aus gesehen, um den Betrag 90° und/oder 240° versetzt sind. Besonders vorteilhaft ist das Anbringen von zwei weiteren Haltenuten, welche um 180° versetzt sind und in den Trennschlitz münden.

Eine weitere bevorzugte Weiterbildung besteht darin, dass die Hohlzylinderwand mit Perforationen versehen ist, z.B. in Form von Kreisbohrungen, Schlitzen oder Langlöchern. Die Perforationen sollten, von der Einbuchtung aus gesehen, um den Betrag 0°, 90°, bzw. 240° angeordnet sein.

Das Zwischenwirbel-Implantat weist für die Anwendung im Lendenwirbelsäulenbereich vorzugsweise eine Höhe im Bereich von 12 bis 23 mm auf; für die Anwendung im Halswirbelsäulenbereich eine solche im Bereich 4,5 bis 12,5 mm.

Für eine Anwendung im Lendenwirbelsäulenbereich weist die äussere Mantelfläche vorzugsweise einen maximalen Abstand von 14 bis 18 mm, von der zentralen Hohlzylinderachse aus gemessen, auf.

Für eine Anwendung im Halswirbelsäulenbereich weist die äussere Mantelfläche vorzugsweise einen maximalen Abstand von 5,5 bis 9,5 mm, von der zentralen Hohlzylinderachse (6) aus gemessen, auf.

Die Erfindung und Weiterbildungen der Erfindung wird im folgenden anhand der teilweise schematischen Darstellungen eines Ausführungsbeispiels noch näher erläutert.

Es zeigen:
Fig. 1 eine perspektivische Darstellung des Zwischenwirbel-Implantats; und
Fig. 2 eine Seitenansicht des Zwischenwirbel-Implantats nach Fig. 1.

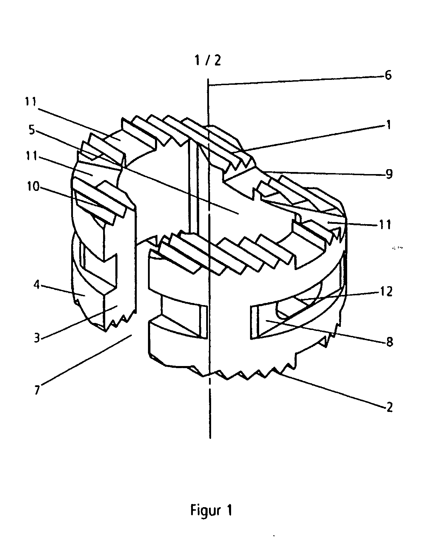

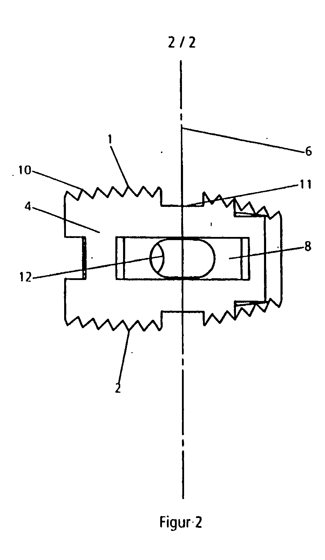

Das in den Fig. 1 und 2 dargestellte Zwischenwirbel-Implantat weist eine hohlzylinderförmige Gestalt auf. Es besitzt eine Deckfläche 1, eine Grundfläche 2, eine Hohlzylinderwand 3 mit einer äusseren Mantelfläche 4 und einer inneren Mantelfläche 5 sowie eine zentrale Hohlzylinderachse 6.

Das Implantat besteht zu mindestens 95 Volumen-Prozent aus einem röntgenstrahlendurchlässigen Material, z.B. PEEK (Gruppe der Polyaryletherketone). Das verwendet Material muss einen E-Modul von 1 bis 20 GPa besitzen. Vorzugsweise beträgt der E-Modul 3 bis 5 GPa.

In der Figuren nicht sichtbar sind drei Marker aus einem röntgenopaken Material (Tantal oder Titan), welche zusammen höchstens 5 Volumenprozente des Zwischenwirbel-Implantats ausmachen. Als Gesamtvolumen (100 %) wird dabei der vom Material des Zwischenwirbel-Implantats eingenommen Rauminhalt verstanden, ohne den vom Implantat umschlossenen Hohlraum.

Die Deck- und Grundflächen 1,2 des Implantats sind mit einer dreidimensionalen Strukturierung 10 versehen, welche aus regelmässig in einem Kreissegment angeordneten Zähnen besteht. Die kreissegmentförmig angeordneten Zähne des Implantats ergeben - zusätzlich zur Keilform des Implantats - eine Wölbung der Deck- und Grundflächen, was wiederum eine optimale Auflage auf der Wirbelkörperendplatte ermöglicht.

Wie aus Fig. 2 gut erkennbar, verlaufen die Deck- und Grundfläche 1,2 keilförmig zueinander und schliessen einen ungefähren Winkel von 10°-20° ein.

Das Implantat weist an der Deckfläche 1 und an der Grundfläche 2 mehrere laterale und anterolaterale Führungsnuten 11 auf, welche gegen die Hohlzylinderachse 6 gerichtet sind. Die Führungsnuten dienen dazu, das Implantat über die Klingen eines Distraktors in den derart aufgespreizten Zwischenwirbelraum einzuführen. Die versetzt angeordneten Nutenpaare erlauben entweder ein laterales, anterolaterales und anteriores Einführen.

Die Führungsnuten 11 sind, von der Hohlzylinderachse 6 aus gesehen, um einen Winkel von 45° ± 15° versetzt angeordnet.

Wie aus Fig. 1 ersichtlich weist die Hohlzylinderwand 3 eine gegen die Hohlzylinderachse 6 gerichtete, von der Deckfläche 1 zur Grundfläche 2 reichende posteriore Einbuchtung 9 auf. Die Hohlzylinderwand 3 weist an ihrer höheren (anterioren) Seite einen parallel zur Hohlzylinderachse 6 verlaufenden, von der Deckfläche 1 zur Grundfläche 2 reichenden Trennschlitz 7 auf, der das Implantat U-förmig gestaltet. Die Einbuchtung 9 ist, von der Hohlzylinderachse 6 aus gesehen, an der dem Trennschlitz 7 gegenüberliegenden Seite der Hohlzylinderwand 3 angebracht.

Die äussere Mantelfläche 4 ist mit zwei lateralen Haltenuten 8 versehen, welche quer zur Hohlzylinderachse 6 verlaufen und den gleichen Abstand zur Deck- und Grund-Fläche 1,2 aufweisen. Von der Einbuchtung 9 aus gesehen, sind die Haltenuten 8 um den Betrag von 90° und 240° versetzt angeordnet.
Im weiteren sind zwei zusätzliche, anteriore Haltenuten 8 vorgesehen, welche um 180° versetzt sind und in den Trennschlitz 7 münden.
Die Funktion der Haltenuten ist es, erstens ein axiales Verdrehen am Implantathalter zu verhindern und zweitens, eine plane Auflagefläche für diese Instrument zu schaffen.

Die Hohlzylinderwand 3 ist mit mindestens einer ovalen Perforation 12 versehen, welche von der Einbuchtung 9 aus gesehen, um den Betrag von 90° versetzt angeordnet ist.

## Patentansprüche

1. Zwischenwirbel-Implantat von hohlzylinderförmiger Gestalt, mit einer Deckfläche (1), einer Grundfläche (2), einer Hohlzylinderwand (3) mit einer äusseren Mantelfläche (4) und einer inneren Mantelfläche (5) sowie einer zentralen Hohlzylinderachse (6), wobei das Implantat zu mindestens 95 Volumen-Prozent aus einem röntgenstrahlendurchlässigen Material besteht,
**dadurch gekennzeichnet, dass**
das Implantat aus einem Material besteht, welches einen E-Modul von 1 bis 20 GPa besitzt.

2. Zwischenwirbel-Implantat nach Anspruch 1, **dadurch gekennzeichnet, dass** das Implantat aus einem Material besteht, welches einen E-Modul von 3 bis 5 GPa besitzt.

3. Zwischenwirbel-Implantat nach Anspruch 1 oder 2, **dadurch gekennzeichnet, dass** das Implantat mindestens einen Marker aus einem röntgenopaken Material aufweist.

4. Zwischenwirbel-Implantat nach Anspruch 3, **dadurch gekennzeichnet, dass** der mindestens eine Marker insgesamt höchstens 5 Volumenprozente des Zwischenwirbel-Implantats ausmacht.

5. Zwischenwirbel-Implantat nach einem der Ansprüche 1 bis 4, **dadurch gekennzeichnet, dass** die Deck- (1) und Grundfläche (2) mit einer dreidimensionalen Strukturierung (10) versehen ist.

6. Zwischenwirbel-Implantat nach Anspruch 5, **dadurch gekennzeichnet, dass** die dreidimensionalen Strukturierung (10) aus Zähnen besteht, vorzugsweise in regelmässiger Anordnung.

7. Zwischenwirbel-Implantat nach Anspruch 5, **dadurch gekennzeichnet, dass** die dreidimensionale Strukturierung (10) aus kreissegmentförmig angeordneten Zähnen besteht.

8. Zwischenwirbel-Implantat nach einem der Ansprüche 1 bis 7, **dadurch gekennzeichnet, dass** die Deck- (1) und Grundfläche (2) keilförmig zueinander verlaufen.

9. Zwischenwirbel-Implantat nach Anspruch 8, **dadurch gekennzeichnet, dass** die Deckfläche (1) zur Grundfläche (2) einen Winkel von 10°-20° einschliesst.

10. Zwischenwirbel-Implantat nach einem der Ansprüche 1 bis 9, **dadurch gekennzeichnet, dass** das röntgenstrahlendurchlässige Material aus der Gruppe der Polyaryletherketone ausgewählt wird.

11. Zwischenwirbel-Implantat nach einem der Ansprüche 1 bis 10 **dadurch gekennzeichnet, dass** die Deckfläche (1) und/oder die Grundfläche (2) mit einer oder mehreren Führungsnuten (11) versehen ist, welche gegen die Hohlzylinderachse (6) gerichtet sind.

12. Zwischenwirbel-Implantat nach Anspruch 11, **dadurch gekennzeichnet, dass** die Führungsnuten (11) von der Hohlzylinderachse (6) aus gesehen, um einen Winkel von 45° ± 15° versetzt angeordnet.

13. Zwischenwirbel-Implantat nach einem der Ansprüche 1 bis 12, **dadurch gekennzeichnet, dass** das Verhältnis DF/FQF zwischen der Deckfläche (1) DF und der freien Querschnittsfläche FQF des von der inneren Mantelfläche (5) umschlossenen Hohlzylinders im Bereich von 0,5 bis 1,6 liegt.

14. Zwischenwirbel-Implantat nach einem der Ansprüche 1 bis 13, **dadurch gekennzeichnet, dass** die Hohlzylinderwand (3) eine gegen die Hohlzylinderachse (6) gerichtete, von der Deckfläche (1) zur Grundfläche (2) reichende Einbuchtung (9) aufweist.

15. Zwischenwirbel-Implantat nach einem der Ansprüche 8 bis 14, **dadurch gekennzeichnet, dass** die Hohlzylinderwand (3) an ihrer höheren Seite einen parallel zur Hohlzylinderachse (6) verlaufenden, von der Deckfläche (1) zur Grundfläche (2) reichenden Trennschlitz (7) aufweist, der das Implantat U-förmig gestaltet.

16. Zwischenwirbel-Implantat nach Anspruch 14 oder 15, **dadurch gekennzeichnet, dass** die Einbuchtung (9), von der Hohlzylinderachse (6) aus gesehen, an der dem Trennschlitz (7) gegenüberliegenden Seite der Hohlzylinderwand (3) angebracht ist.

17. Zwischenwirbel-Implantat nach einem der Ansprüche 1 bis 16, **dadurch gekennzeichnet, dass** die äussere Mantelfläche (4) mit einer oder mehreren Haltenuten (8) versehen ist, welche quer zur Hohlzylinderachse (6) verlaufen und den gleichen Abstand zur Deck- (1) und Grund-fläche (2) aufweisen.

18. Zwischenwirbel-Implantat nach Anspruch 17, **dadurch gekennzeichnet, dass** mehrere Haltenuten (8) vorgesehen sind, welche von der Einbuchtung (9) aus gesehen, um den Betrag 90° und/oder 240° versetzt sind.

19. Zwischenwirbel-Implantat nach Anspruch 17 oder 18, **dadurch gekennzeichnet, dass** zwei weitere Haltenuten (8) vorgesehen sind, welche um 180° versetzt sind und in den Trennschlitz (7) münden.

20. Zwischenwirbel-Implantat nach einem der Ansprüche 1 bis 19, **dadurch gekennzeichnet, dass** die Hohlzylinderwand (3) mit Perforationen (12) versehen ist.

21. Zwischenwirbel-Implantat nach Anspruch 20, **dadurch gekennzeichnet, dass** die Perforationen (12) als Kreisbohrung, Schlitz oder Langloch ausgebildet sind.

22. Zwischenwirbel-Implantat nach Anspruch 21, **dadurch gekennzeichnet, dass** die Perforationen (12), von der Einbuchtung (9) aus gesehen, um den Betrag 0°, 90°, bzw. 240° angeordnet sind.

23. Zwischenwirbel-Implantat nach einem der Ansprüche 1 bis 22, **dadurch gekennzeichnet, dass** seine Höhe im Bereich von 12 bis 23 mm liegt.

24. Zwischenwirbel-Implantat nach einem der Ansprüche 1 bis 22, **dadurch gekennzeichnet, dass** seine Höhe im Bereich von 4,5 bis 12,5 mm liegt.

25. Zwischenwirbel-Implantat nach einem der Ansprüche 1 bis 24, **dadurch gekennzeichnet, dass** die äussere Mantelfläche (4) einen maximalen Abstand von 14 bis 18 mm, von der zentralen Hohlzylinderachse (6) aus gemessen, aufweist.

26. Zwischenwirbel-Implantat nach einem der Ansprüche 1 bis 24, **dadurch gekennzeichnet, dass** die äussere Mantelfläche (4) einen maximalen Abstand von 5,5 bis 9,5 mm, von der zentralen Hohlzylinderachse (6) aus gemessen, aufweist.

27. Zwischenwirbel-Implantat nach einem der Ansprüche 1 bis 26, **dadurch gekennzeichnet, dass** das röntgenstrahlendurchlässige Material aus der Gruppe der Polyetheretherketone (PEEK), ultrahochmolekularen Polyethylene (UHMWPE) oder Polysulfone (PSU) ausgewählt wird.

## Claims

1. An intervertebral implant shaped in the form of a hollow cylinder, including a cover face (1), a base face (2), a hollow cylinder wall (3) with an outer lateral area (4) and an inner lateral area (5), as well as a hollow cylinder central axis (6), the implant consisting at at least 95 percent by volume of a radiolucent material,
**characterised in that**
the implant consists of a material which has a modulus of elasticity of between 1 and 20 GPa.

2. An intervertebral implant as claimed in claim 1, **characterised in that** the implant is made of a material having a modulus of elasticity of between 3 and 5 GPa.

3. An intervertebral implant as claimed in claim 1 or 2, **characterised in that** the implant is provided with at least one marker consisting of a radiopaque material.

4. An intervertebral implant as claimed in claim 3, **characterised in that** the at least one marker makes up in total at most 5 percent by volume of said intervertebral implant.

5. An intervertebral implant as claimed in any of the claims 1 to 4, **characterised in that** the cover face (1) and the base face (2) are provided with a three-dimensionally structured surface (10).

6. An intervertebral implant as claimed in claim 5, **characterised in that** the three-dimensionally structured surface (10) consists of teeth, preferably arranged in a regular pattern.

7. An intervertebral implant as claimed in claim 5, **characterised in that** the three-dimensionally structured surface (10) consists of teeth arranged in the form of a segment of a circle.

8. An intervertebral implant as claimed in any of the claims 1 to 7, **characterised in that** the cover face (1) and the base face (2) are arranged in a wedge-shaped, tapered form relative to each other.

9. An intervertebral implant as claimed in claim 8, **characterised in that** the cover face (1) and the base face (2) form an angle of between 10 and 20 degrees.

10. An intervertebral implant as claimed in any of the claims 1 to 9, **characterised in that** the radiolucent material is selected from the group of polyaryletherketones.

11. An intervertebral implant as claimed in any of the claims 1 to 10, **characterised in that** the cover face (1) and/or the base face (2) are provided with one or several guide notches (11) which are oriented towards the hollow cylinder axis (6).

12. An intervertebral implant as claimed in claim 11, **characterised in that** the guide notches (11) are offset by an angle of 45 degrees ± 15 degrees as seen from the hollow cylinder axis (6).

13. An intervertebral implant as claimed in any of the claims 1 to 12, **characterised in that** the ratio CF/FCA between the cover face (1) CF and the free cross-sectional area FCA of the hollow cylinder defined by the internal lateral area (5) should suitably be in the range of between 0.5 and 1.6.

14. An intervertebral implant as claimed in any of the claims 1 to 13, **characterised in that** the hollow cylinder wall (3) is provided with a recess (9) oriented towards the hollow cylinder axis (6) and extending from the cover face (1) to the base face (2).

15. An intervertebral implant as claimed in any of the claims 8 to 14, **characterised in that** on its higher side the hollow cylinder wall (3) has a separating slot (7) extending parallel to the hollow cylinder axis (6) and reaching from the cover face (1) to the base face (2), so as to give a U-shaped form to the implant.

16. An intervertebral implant as claimed in claim 14 or 15, **characterised in that** the recess (9) is arranged on the side of the hollow cylinder wall (3) situated opposite to the separating slot (7) as seen from the hollow cylinder axis (6).

17. An intervertebral implant as claimed in any of the claims 1 to 16, **characterised in that** the outer lateral area (4) is provided with one or several retaining notches (8) extending at a right angle to the hollow cylinder axis (6) and located equidistant from the cover face (1) and the base face (2).

18. An intervertebral implant as claimed in claim 17, **characterised in that** several retaining notches (8) are included which are offset by an amount of 90 degrees and/or 240 degrees as seen from the recess (9).

19. An intervertebral implant as claimed in claim 17 or 18, **characterised in that** two further retaining notches (8) are included which are offset by 180 degrees and lead to the separating slot (7).

20. An intervertebral implant as claimed in any of the claims 1 to 19, **characterised in that** the hollow cylinder wall (3) is provided with perforations (12).

21. An intervertebral implant as claimed in claim 20, **characterised in that** the perforations (12) are shaped in the form of circular bores, slots, or elongate holes.

22. An intervertebral implant as claimed in claim 21, **characterised in that** the perforations (12) are offset by an amount of 0 degrees, 90 degrees, and/or 240 degrees, as seen from the recess (9).

23. An intervertebral implant as claimed in any of the claims 1 to 22, **characterised in that** its height is in the range of between 12 and 23 mm.

24. An intervertebral implant as claimed in any of the claims 1 to 22, **characterised in that** its height is in the range of between 4.5 and 12.5 mm.

25. An intervertebral implant as claimed in any of the claims 1 to 24, **characterised in that** the outer lateral area (4) extends at a maximum distance of between 14 and 18 mm, measured from the hollow cylinder central axis (6).

26. An intervertebral implant as claimed in any of the claims 1 to 24, **characterised in that** the outer lateral area (4) extends at a maximum distance of between 5.5 and 9.5 mm, measured from the hollow cylinder central axis (6).

27. An intervertebral implant as claimed in any of the claims 1 to 26, **characterised in that** the radiolucent material is selected from among the groups of polyetheretherketones (PEEK), ultra-high molecular weight polyethylenes (UHMWPE) or polysulfones (PSU).

## Revendications

1. Implant intervertébral de forme cylindrique creuse, avec une face de dessus (1), une face de dessous (2), une paroi de cylindre creux (3) avec une surface latéral externe (4) et une surface latéral interne (5) ainsi qu'un axe central de cylindre creux (6), au moins 95 pour cent volumétrique de l'implant étant composés d'un matériau transparent aux rayons X,
**caractérisé en ce que**
l'implant est composé d'un matériau qui possède un coefficient d'élasticité entre 1 et 20 GPa.

2. Implant intervertébral selon la revendication 1, **caractérisé en ce que** l'implant est composé d'un matériau qui possède un coefficient d'élasticité entre 3 et 5 GPa.

3. Implant intervertébral selon la revendication 1 ou 2, **caractérisé en ce que** l'implant présente au moins un marqueur composé d'un matériau opaque aux rayons X.

4. Implant intervertébral selon la revendication 3, **caractérisé en ce que** ledit marqueur représente en tout au plus 5 pour cent volumétrique de l'implant intervertébral.

5. Implant intervertébral selon une des revendications 1 à 4, **caractérisé en ce que** les faces de dessus (1) et de dessous (2) sont munies d'une structure tridimensionnelle (10).

6. Implant intervertébral selon la revendication 5, **caractérisé en ce que** la structure tridimensionnelle (10) est composée de dents, disposées de préférence de façon régulière.

7. Implant intervertébral selon la revendication 5, **caractérisé en ce que** la structure tridimensionnelle (10) est composée de dents disposées en segments de cercle.

8. Implant intervertébral selon une des revendications 1 à 7, **caractérisé en ce que** les faces de dessus (1) et de dessous (2) se rejoignent de façon cunéiforme.

9. Implant intervertébral selon la revendication 8, **caractérisé en ce que** la face de dessus (1) forme un angle de 10° à 20° avec la face de dessous (2).

10. Implant intervertébral selon une des revendications 1 à 9, **caractérisé en ce que** le matériau transparent aux rayons X appartient au groupe des polyarytéthercétones.

11. Implant intervertébral selon une des revendications 1 à 10, **caractérisé en ce que** la face de dessus (1) et/ou la face de dessous (2) est munie d'une ou plusieurs rainures de guidage (11) orientées vers l'axe de cylindre creux (6).

12. Implant intervertébral selon la revendication 11, **caractérisé en ce que** les rainures de guidage (11) sont décalées d'un angle de 45° ± 15°, vu depuis l'axe de cylindre creux (6).

13. Implant intervertébral selon une des revendications 1 à 12, **caractérisé en ce que** le rapport DF/FQF entre la face de dessus (1) DF et la superficie de section libre FQF du cylindre creux enveloppé par la surface latérale interne (5) est compris entre 0,5 et 1,6.

14. Implant intervertébral selon une des revendications 1 à 13, **caractérisé en ce que** la paroi de cylindre creux (3) présente un évidement (9) orienté vers l'axe de cylindre creux (6) et allant de la face de dessus (1) à la face de dessous (2).

15. Implant intervertébral selon une des revendications 8 à 14, **caractérisé en ce que** la paroi de cylindre creux (3) présente, sur sa face la plus haute, une fente de séparation (7) parallèle à l'axe de cylindre creux (6), allant de la face de dessus (1) à la face de dessous (2) et donnant la forme d'un U à l'implant.

16. Implant intervertébral selon la revendication 14 ou 15, **caractérisé en ce que**, vu depuis l'axe de cylindre creux (6), l'évidement (9) est placé sur le côté de la paroi de cylindre creux (3) opposé à la fente de séparation (7).

17. Implant intervertébral selon une des revendications 1 à 16, **caractérisé en ce que** la surface latérale externe (4) est munie d'une ou plusieurs rainures de retenue (8), perpendiculaires à l'axe de cylindre creux (6) et situées à égale distance des faces de dessus (1) et de dessous (2).

18. Implant intervertébral selon la revendication 17, **caractérisé en ce que** l'on prévoit plusieurs rainures de retenue (8) qui, vu depuis l'évidement (9), sont décalées de 90° et/ou 240°.

19. Implant intervertébral selon la revendication 17 ou 18, **caractérisé en ce que** l'on prévoit deux autres rainures de retenue (8), décalées de 180° et débouchant dans la fente de séparation (7).

20. Implant intervertébral selon une des revendications 1 à 19, **caractérisé en ce que** la paroi du cylindre creux (3) est munie de perforations (12).

21. Implant intervertébral selon la revendication 20, **caractérisé en ce que** les perforations (12) ont la forme d'un alésage circulaire, d'une fente ou d'un trou oblong.

22. Implant intervertébral selon la revendication 21, **caractérisé en ce que**, vu depuis l'évidement (9), les perforations (12) sont placées à 0°, 90° et 240°.

23. Implant intervertébral selon une des revendications 1 à 22, **caractérisé en ce que** sa hauteur est comprise entre 12 et 23 mm.

24. Implant intervertébral selon une des revendications 1 à 22, **caractérisé en ce que** sa hauteur est comprise entre 4,5 et 12,5 mm.

25. Implant intervertébral selon une des revendications 1 à 24, **caractérisé en ce que** la surface latérale externe (4) présente un écart maximal de 14 à 18 mm, mesuré depuis l'axe central du cylindre creux (6).

26. Implant intervertébral selon une des revendications 1 à 24, **caractérisé en ce que** la surface latéral externe (4) présente un écart maximal de 5,5 à 9,5 mm, mesuré depuis l'axe central du cylindre creux (6).

27. Implant intervertébral selon une des revendications 1 à 26, **caractérisé en ce que** le matériau transparent aux rayons X appartient au groupe des polyétheréthercétones (PEEK), des polyéthylènes de poids moléculaire ultra-élevé (UHMWPE) ou des polysulfones (PSU).
